(19) Europäisches Patentamt
European Patent Office
Office européen des brevets

(11) Publication number: **0 255 485**
**A2**

(12) # EUROPEAN PATENT APPLICATION

(21) Application number: 87810423.1

(22) Date of filing: 27.07.87

(51) Int. Cl.$^4$: **A 61 K 47/00**
A 61 L 15/03

(30) Priority: 01.08.86 US 893119

(43) Date of publication of application:
03.02.88 Bulletin 88/05

(84) Designated Contracting States:
AT BE CH DE ES FR GB GR IT LI LU NL SE

(71) Applicant: WARNER-LAMBERT COMPANY
201 Tabor Road
Morris Plains New Jersey 07950 (US)

(72) Inventor: Mahjour, Majid
82 Koclas Drive
Netcong New Jersey 07857 (US)

Fawzi, Mahdi B.
11 Tiberline Drive
Flanders New Jersey 07836 (US)

Iyer, Uma
7 Florie Farm Road
Mendham New Jersey 07945 (US)

(74) Representative: Silbiger, Jakob, Dr.
c/o CAPSUGEL AG Münchensteinerstrasse 41 Postfach
CH-4002 Basel (CH)

(54) **Transdermal compositions.**

(57) The penetration of certain analgesic, cardiotonic, and/or bronchodilating agents through living skin is enhanced by the use of a ternary vehicle which contains a fatty component and a co-solvent system.

EP 0 255 485 A2

**Description**

TRANSDERMAL COMPOSITIONS

The administration of drugs via oral routes is often associated with a variety of problems, e.g., gastro-intestinal side effects, unpleasant taste and/or odor, and the like.

Recent studies have shown that the transdermal administration of drugs offers a viable alternative to oral dosage forms. However, transdermal delivery systems are not always efficacious due to such factors as the failure of the drug to adequately penetrate the skin and enter the body for metabolism therein.

It has been discussed that the penetration of certain drugs through living cutaneous membranes can be augmented by the use of a ternary penetration enhancement system.

In one preferred embodiment, a narcotic analgesic such as fentanyl, morphine, hydromorphone. or oxymorphone is combined with linoleic acid. The linoleic acid-containing formulation is absorbed dramatically faster than a preparation that does not contain linoleic acid. When triacetin and propylene glycol and/or PEG 400 are added to these formulations, transdermal permeation is increased by a factor of 50 to 100, compared to formulations devoid of linoleic acid.

In another embodiment, cardiotonic compounds, such as those of the formula:

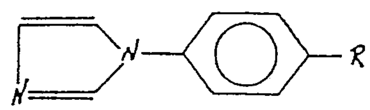

wherein R is 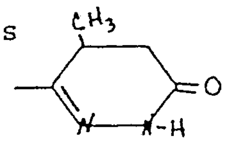 or 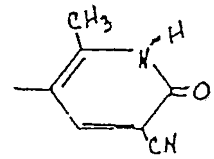

or their pharmaceutically acceptable salts are formulated with linoleic acid.

In yet another embodiment, procaterol, a bronchodilator, is combined with the instant ternary system. The transdermal permeation of such formulations is enhanced over those lacking linoleic acid. In addition, compositions containing these cardiotonic agents, linoleic acid and one or more of triacetin and propylene glycol, show markedly improved penetration of the cardiotonic agent(s) over conventional compositions containing only solvent (e.g., propylene glycol).

The invention deals with novel transdermal formulations for the delivery of analgesic, cardiotonic, and/or bronchodilating agents. It also is concerned with methods of administering those compositions topically to living cutaneous membranes. The instant compositions can be delivered buccally, nasally, transdermally, or via any other means through which the agent(s) enters the bloodstream by transversing a lining membrane.

The compositions and methods of the invention have several advantages over prior art delivery systems. Since the instant system permits delivery via buccal, rectal, mucosal, nasal and dermal membranes, the drug can enter the bloodstream without entering the gastrointestinal tract. Transmembrane dosage forms generally do not produce the side effects, such as nausea and the like, which are often associated with the oral administration of drugs.

Furthermore. since the instant compositions need not be administered via injection, the unpleasantness of that type of delivery is avoided.

In general, it has been found that administration via penetration of the drug across a suitable membrane, e.g., the cutaneous barrier, is superior to other routes of administration, especially in terms of ease of administration and attainment of sustained release.

Other aspects and advantages of this invention will be apparent from the following description of the invention.

The invention is as described in the claims. It can further be described as follows.

The compositions of the invention contain at least one analgesic and/or at least one cardiotonic agent and/or one bronchodilator in a vehicle system suited for topical administration of the compositions.

Weight percentages are based on total composition weight unless stated otherwise.

The compositions of the invention will generally contain an active ingredient, e.g., a drug, and a vehicle comprising (a) an essential fatty acid, ester, or alcohol as a primary component and (b) co-solvents as a secondary component.

The active ingredients employed in the compositions of the invention are selected from a small group of compounds. They are generally analgesics, bronchodilators, and cardiotonics.

Useful analgesics are drugs of the morphine family, their pharmaceutically acceptable derivatives or synthetic analgesics such as fentanyl. The preferred drugs are morphine, hydromorphone, oxymorphone and fentanyl. The most preferred drugs are oxymorphone and fentanyl.

Useful cardiotonics preferably include recently discovered compounds conforming to the formulas set out below:

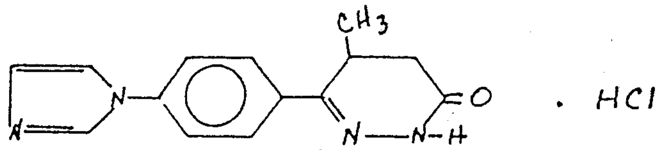

(I)

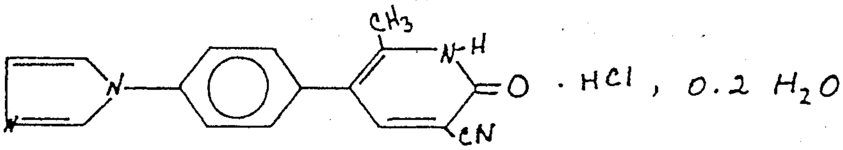

(II)

Compounds of formula (I) are highly preferred. Other pharmaceutically acceptable forms of these compounds can be employed.

When a bronchodilator is employed, it is preferred that it be of the beta-blocker type. Procaterol is a preferred bronchodilator.

The use of combinations of these compounds, i.e., I and II together. as well as combinations containing one or more of the types of drugs discussed above, is contemplated.

The pharmaceutically acceptable derivatives, e.g. hydrochloride salts, of any of the drugs discussed above, can be used.

The vehicle system used in the instant formulations will comprise three or more substances which aid in the transdermal penetration of the active ingredient(s) so that it is absorbed into the bloodstream.

The vehicle must contain a primary fatty component and a secondary co-solvent system as component.

The primary component is at least one essential fatty acid or a structurally related analog thereof. Thus, esters and alcohols containing essential fatty moieties are contemplated. Useful esters are the methyl, ethyl, and propyl esters of fatty acids. Useful alcohols are mono- and poly-hydroxy analogs of essential fatty acids, e.g. linoleyl alcohol. Mixtures are operable.

The preferred fatty component is selected from oleic, linolenic, and linoleic acid(s), their esters and alcohols.. Linoleic acid, itself, is highly preferred.

The use of linoleic acid to enhance the penetration of anti-inflammatory and analgesic agents has been described in Kokai Tokkyo Kobo, "Topical Anti-inflammatory and Analgesic Agents," Taisho Pharmaceutics Co., Ltd., Japan, 81, 110, 614(cl. A61K31/135) September 1, 1981.

In addition, several studies have been made on the function of linoleic acid, an essential fatty acid, in skin. Two of these are: C. Prottey, "Investigation of Functions of Essential Fatty Acids in the Skin", British Journal of Dermatology, (1977). 97, 29; and E. O. Butcher, "The Penetration of Fat and Fatty Acid into the Skin of the Rat", J. Investig. Dermatol. (1953), 43-48.

The above-mentioned disclosures are hereby incorporated by reference.

The mechanism by which the linoleic acid, alcohol, or ester enhances the penetration of the instant combinations is not clearly understood. However, it is believed that the linoleic component acts as a structural element in the cell phospholipids of the selected cutaneous membrane, e.g., nasal or dermal.

The co-solvent or secondary component of the penetrant system of the invention does not contain a fatty substance. Generally, this component comprises one or more solvents for the bioaffecting agent. Useful solvents are generally mono- or poly-hydroxyl-containing compounds and compounds containing one or more ester groups.

The secondary component is selected, principally, for its compatibility with the active ingredient or drug. For compositions based on the specific drugs discussed herein, a combination of one or more ester and one or more alcoholic solvents is preferred.

The alcoholic portion of the secondary component is generally a $C_{2-4}$ alkanol or glycol or polymeric form thereof. Thus ethanol, propanol, n-butanol, sec-butanol, t-butanol, propylene glycol, polyethylene glycol 400, and the like are operable. Ethanol and propylene glycol are preferred alcoholic solvents. Mixtures are operable.

The ester portion of the secondary component is generally a $C_2$ glycerol ester, e.g., triacetin, glyceryl triacetate, and the like.

Preferred glycerol esters are diacetin and triacetin. Triacetin is highly preferred.

The relative proportions of each of the ingredients discussed above are present in the subject compositions as set out in Table I.

Table I

Table I: Typical Proportions in which Ingredients will be used in the Compositions of the Invention

| | Weight Percentage Range | | |
|---|---|---|---|
| | Broad | Highly Preferred | Preferred |
| Active Ingredient | 0.1-10 | 1-5 | 2 |
| Vehicle | | | |
| Fatty Component | 2-30 | 5-25 | 20 |
| Secondary Component(s) | | | |
| Alcohol(s) or glycol(s) | 1-99 | 10-50 | 30 |
| Ester(s) | 1-99 | 30-60 | 50 |

Generally, the weight ratio of alcoholic to ester components will range from about 0.05:1 to about 1:1. The use of excipients and other conventional additives is contemplated. The use of drugs other than those specifically discussed above is also contemplated.

Example I

Assay Technique

The amount of narcotic agent(s) in the skin diffusion samples studied was quantified using the following high pressure liquid chromatography (HPLC) assay. An HPLC method, which employed an isocratic mobile phase, a CN column and a UV detector, was used for the quantitation of all three narcotic compounds present in the skin diffusion samples. Over a concentration range of 3 to 50 ug/ml, the system is reproducible and linear. The retention times for morphine, oxymorphone, and hydromorphone are 8.3, 8.7 and 9.7 minutes, respectively, with this system.

Mobile Phase:

A 2:8 mixture of acetonitrile and a solution of 0.05 M ammonium phosphate adjusted to pH 3 with phosphoric acid and diluted 1 to 16 with water.

Detection Wavelength:

220 nm

Stationary Phase:

Dupont Zorbax CN 4.6 mm (internal diameter) × 25 cm (length) column.

Recorder:

Spectra Physics SP4270 Computing Integrator

Receiver Solution:

Isotonic Citrate/Phosphate Buffer PH 4.5

Diffusion Studies

Skin Preparation:

Male hairless mice, approximately 6-8 weeks old, were sacrificed by cervical dislocation. Square sections of skin (about 3-5 $cm^2$) were excised from the animals with surgical scissors. The skin was mounted on Franz® diffusion cells with the dermal side facing the receiver compartment, and leached with saline for 2 hours before use. Temperature was maintained at 37°C during the experiment.

Table II shows the permeability data for the selected narcotic analgesics at concentrations of 0.5%. The permeation of the compounds from the mixture of propylene glycol triacetin was negligible. The concentration of each analgesic can be increased to 3% or 4% in the donor solution and corresponding increases in flux can be expected. Oxymorphone, for example, at a loading concentration of 0.5% had a flux of 66.6 ug/$cm^2$/hr which is equivalent to the delivery of about 16 mg/day from a 10 $cm^2$ patch through hairless mouse skin and

about 3.2 mg/day through human skin, assuming 1/5 the permeability. A usual dose for oxymorphone and hydromorphone is about 6 to 10 mg per day in humans. By increasing the loading concentration to 2%, a four-fold increase in flux can be expected; therefore, about 12.8 mg/day would be delivered from a 10 $cm^2$ patch in humans.

Table II: Effect of linoleic acid concentration on the permeation of the selected narcotic analgesics, using compositions containing 0.5 wt % analgesic

| Formulation | Compound | Conc. mg/ml | Flux ug/$cm^2$/hr | PX$10^6$ cm/sec | Lag Time hr |
|---|---|---|---|---|---|
| LA:PG:TA 20:30:50 | MO | 5.3 | 57.5 | 3.0 | 3.4 |
| | OX | 5.3 | 66.6 | 3.5 | 3.5 |
| | HM | 5.3 | 54.5 | 2.9 | 3.4 |
| LA:PG:TA 10:30:60 | MO | 5.4 | 51.6 | 2.7 | 4.2 |
| | OX | 5.4 | 51.5 | 2.7 | 4.1 |
| | HM | 5.4 | 51.5 | 2.7 | 4.1 |
| LA:PG:TA 5:30:65 | MO | 5.5 | 43.1 | 2.2 | 7.7 |
| | OX | 5.5 | 40.0 | 2.0 | 7.6 |
| | HM | 5.5 | 44.4 | 2.2 | 7.7 |

LA = Linoleic Acid PG = Propylene Glycol TA = Triacetin
OX = Oxymorphone HM = Hydromorphone MO = Morphine

The numbers shown in the formulation column correspond to the number of grams of each solvent used. Thus a 20:30:50 ratio of LA:PG:TA contained 20 grams linoleic acid, 30 grams propylene glycol and 50 grams triacetin.

Example II

Using procedures similar to that employed in Example I, the premeation of compound I from vehicles containing 0, 10, 40, 80, or 100% linoleic acid in propylene glycol was determined (see Table III). Then diffusion studies were conducted on compositions which contained lesser amounts of propylene glycol than the above formulations.

Table III: Effect of Linoleic Acid Concentration in Propylene Glycol on the Premeation of Compound I Base (expressed as hydrochloride salt of Compound I)

| % Linoleic Acid | Conc'n. (mg/ml) | Flux (ug/cm²/h) | Permeability Coefficient (P) (cm/sec x $10^7$) | $\frac{P}{P_x}$* | Lag Time (h) |
|---|---|---|---|---|---|
| 0 | 26.38 | 0.4 | 0.042 | 1 | 6.2 |
| 10 | 43 | 492 | 31.8 | 755 | 1.75 |
| 40 | 43 | 337.3 | 21.8 | 517 | 1.45 |
| 80 | 43 | 148.5 | 9.6 | 227 | 1.29 |
| 100 | 31.5 | 24.5 | 2.2 | 51 | 5.35 |

$P_x$ *Permeability coefficient of the drug from propylene glycol solution

Compound I is the compound of Formula I, supra.

Example III:

Using similar procedures, the results of replacing 60% of the propylene glycol in the formulations with triacetin or 100% with tetraglycol are shown in Table IV. Both formulations could provide the daily therapeutic dose of 1 to 2 mg in man, assuming human skin to be 5 times less permeable than mouse skin.

Table IV: Effect of Linoleic Acid on Permeation of Compound I Base in Vehicles Containing Triacetin or Tetraglycol.

| Vehicle | Conc'n. (mg/ml) | Flux (ug/cm²/h) | Permeability Coefficient (P) (cm/sec x $10^7$) | $\frac{P}{P_x}$* | Lag Time (h) |
|---|---|---|---|---|---|
| LA:TA:PG 20:50:30 | 21.57 | 130.14 | 16.8 | 400 | 1.91 |
| LA:TG 20:80 | 20.09 | 186.69 | 25.8 | 614 | 5.58 |

*Permeability coefficient of the drug from propylene glycol solution

TG = tetraglycol LA = linoleic acid

PG = propylene glycol TA = triacetin

The flux of the base from a mixture of PEG 400 and linoleic acid (20:80 w/w) was 114.3 ug/cm²/h with a permeability coefficient of 7.6 × $10^{-6}$ cm/sec and a lag time of about 3.5 hours.

Example IV

Using similar procedures to those used above tests were conducted to ascertain the permeation of procaterol from a solvent system containing linoleic acid, triacetin, and propylene glycol. The results for that system are given in Table V.

Table V: Permeation of Procaterol from Ternary (Linoleic Acid: Triacetin:Propylene glycol) System

| Vehicle | Procaterol Conc.* (mg/ml) | Flux ($ug/cm^2/h$) | P (cm/sec x $10^7$) | Lag Time (h) |
|---|---|---|---|---|
| LA:TA:PG 20:50:30 | 40 | 158.6 | 11 | 1.26 |

*Procaterol concentration and flux values are expressed in terms of the hydrochloride salt.

Example V

Using procedures similar to those described above, data was generated to show the permeation of procaterol using formulations containing linoleic and (LA), triethyl citrate (TEC) and propylene glycol (PG). The results are given in Table VI.

TABLE VI: Permeation of Procaterol from Formulations Containing Linoleic Acid, Triethyl Citrate, and Propylene Glycol.

| Vehicle LA:TEC:PG | Drug Conc.* mg/ml | Flux* ($ug/cm^2/h$) | P cm/sec x $10^7$ | Lag Time (h) |
|---|---|---|---|---|
| 10:80:10 | 16.9 | 32.0 | 5.26 | 14.7 |
| 10:70:20 | 23.03 | 33.5 | 4.04 | 14.7 |
| 20:60:20 | 19.35 | 46.0 | 6.60 | 4.9 |
| 20:50:30 | 44.7 | 65.8 | 4.10 | 7.3 |
| 20:50:30 | 4.32 | 8.5 | 5.47 | 11.2 |

*Procaterol concentration and flux values are expressed in terms of the hydrochloride salt.

Triethyl citrate is miscible with linoleic acid in all proportions.

Example VI

The permeation of procaterol hydrochloride through hairless mouse skin was studied using various concentrations of linoleic and (LA), propylene glycol (PG) and triacetin (TA).

The permeability data for procaterol hydrochloride through hairless mouse skin are shown in Table VII. A flux value of about 14 $ug/cm^2/h$ or 3.36 $mg/day/10\ cm^2$ was obtained with the formulation containing LA:PG:TA (20:30:50).

Assuming the permeability of human skin to be 1/5 that of mouse skin, a daily flux of 0.68 mg can be achieved in man. The maximum daily dose in man is approximated to be 0.2 mg.

Permeability data for the base converted from the salt are shown in Table VIII. Higher flux values were obtained with the base. In converting the salt of procaterol to the base, Method II (described below) is preferred to achieve a more complete conversion and, consequently, higher flux values.

TABLE VII: Permeation of Procaterol Hydrochloride through Hairless Mouse Skin

| Solvent System | Conc.* (mg/ml) | Flux ($ug/cm^2/h$) | P ($cm/sec \times 10^7$) | Lag Time (h) |
|---|---|---|---|---|
| LA:PG:TA 20:30:50 | 5 | 14 | 7.7 | 4.0 |
| PG | 5 | ** | -- | -- |

* approximate concentration
** lower than HPLC sensitivity

TABLE VIII: Permeation of Procaterol Hydrochloride, Converted to Base,* using Various Systems

| Ratio of LA:TA:PG | Flux+ $uq/cm^2/h$ | Lag time (h) |
|---|---|---|
| 5:85:10 | 23.1 | 5.5 |
| 10:80:10 | 44.2 | 4.8 |
| 15:75:10 | 53.2 | 4.05 |
| 20:75:5 | 33.9 | 4.8 |
| 20:50:30 | 56.3 | 4.4 |

*Due to incomplete conversion of the salt to the base, the concentration of procaterol in these formulations could not be measured.
+Flux values are expressed in terms of procaterol hydrochloride.

A typical study made using Method II involved:
A weighed amount of procaterol hydrochloride was dissolved in about 15 times its weight of methanol. Diethanolamine, equimolar to procaterol, was then added to the solution. After sonicating for three minutes, the solution became clear and yellow in color. The methanolic solution was evaporated to dryness under nitrogen and the residue placed under vacuum for twenty minutes. The transdermal solvent system was then added to the residue and sonicated for 10 minutes.

Other amines or amino acids (e.g. arginine) can be used to covert the salt to base.

Example VII

Additional tests were performed to evaluate the effect of other fatty acids and their esters on the permeation of procaterol. Other fatty acids such as oleic and linolenic acid, in combination with propylene glycol, yielded permeability data comparable to that seen with linoleic acid. As shown in Table IX, the fatty acid ester, ethyl linoleate, in combination with propylene glycol, yielded a high permeability coefficient. However, it should be noted that the solvent system was poorly miscible and had a low capacity for dissolving procaterol.

TABLE IX: Effect of Some Fatty Acids and Their Esters on the Permeation of Procaterol

| Vehicle | Drug Conc.* mg/ml | Flux (ug/cm$^2$/h) | P (cm/sec x $10^7$) | Lag Time (h) |
|---|---|---|---|---|
| Ethyl Linoleate: PG:TA 10:5:85 | 0.855 | 9.60 | 31.2 | 6 |
| Ethyl Linoleate:TG 10:90 | 12.29 | 36.27 | 8.2 | 8 |
| Oleic Acid:PG:TA 20:30:50 | 15.89 | 171.76 | 30 | 1.8 |
| LNA:PG:TA 20:15:65 | 8.15 | 76.27 | 26 | 2 |

*Flux values and drug concentrations are expressed as procaterol hydrochloride.

PG = Propylene Glycol

TA = Triacetin

TG = Tetraglycol

LNA = Linolenic Acid

Reasonable variations, such as those which would occur to a skilled artisan, can be made herein without departing from the scope of the invention.

**Claims**

1. A transdermal formulation containing:
   (a) at least one drug, and
   (b) a vehicle comprising
      (1) an essential fatty component, and
      (2) a secondary component which contains two solvents for the drug, which solvents comprise at least one ester and at least one alcohol or glycol.
2. The formulation of claim 1 wherein (1) is selected from the group consisting of linoleic acid, linoleyl alcohol and a $C_{1-3}$ ester of linoleic acid.
3. The formulation of Claim 2 wherein (2) contains triacetin, at least one of propylene glycol and polyethylene glycol.
4. A method of administering an analgesic agent comprising contacting the formulation of Claim 1 with a living cutaneous membrane.
5. A method of administering a cardiotonic agent comprising contacting the composition of Claim 3 with a living cutaneous membrane.
6. A method 6 administering a bronchodilating agent comprising contacting the composition of Claim 3 with a living cutaneous membrane.